# EUROPEAN PATENT APPLICATION

(11) **EP 3 415 074 A2**
(43) Date of publication of application: **19.12.2018**
(21) Application number: 18174253.7
(22) Date of filing: 25.05.2018
(51) Int. Cl.: A61B 1/00, A61B 1/01, A61B 34/20, A61B 5/06, A61B 1/267

(54) **SURGICAL APPARATUS INCLUDING ELASTOMERIC SHEATH**

(30) Priority: 26.05.2017 US 201715606344
(71) Applicant: Covidien LP, Mansfield, MA 02048 (US)
(72) Inventor: KRIMSKY, William, Forest Hill, MD 21050 (US); STOPEK, Joshua, Minneapolis, MN 55419 (US)
(74) Representative: Maschio, Antonio

(57) **Abstract**

Surgical apparatus (100) for navigating and guiding surgical tools and instruments to a target within a patient's body are described. One such apparatus includes an elastomeric sheath (106) having a first lumen (122) and a second lumen (124). The first lumen is configured to receive an endoscope (108) and the second lumen is configured to receive a surgical tool (110). The elastomeric sheath (106) further includes a navigation sensor (134) disposed on a distal portion (118) of the elastomeric sheath (106). The navigation sensor (134) is configured to be detectable in an electromagnetic field and aid in guiding the elastomeric sheath and endoscope, or more particularly a bronchoscope, through the network of a patient's airways. Other embodiments of the apparatus may include balloon anchor (138) disposed on the sheath (106) or an additional collapsible lumen (140) disposed on the sheath (106) and configured to provide an additional passageway (142) for a surgical tool or device.

## Description

### BACKGROUND

### 1. Technical Field

The present disclosure generally relates to a sheath configured to receive an endoscope and a surgical tool. In particular, the present disclosure relates to a sheath including a navigational sensor and two or more lumens for use with an endoscope, or more particularly a bronchoscope, and additional surgical tools.

### 2. Background of Related Art

A common interventional procedure in the field of pulmonary medicine is bronchoscopy, in which a bronchoscope is inserted into the airways through the patient's nose or mouth. Bronchoscopes are routinely used in the diagnosis and treatment of lung conditions, such as, lung cancer, airway stenosis, emphysema, etc.

The structure of a bronchoscope generally includes a handle and a long, thin, flexible tube extending from the handle. The tube typically defines a lumen or working channel therethrough for the insertion of instruments, such as, for example, diagnostic tools (e.g., biopsy tools, etc.) or therapeutic tools (e.g., lasers, cryogenic probes, radio frequency probes, microwave tissue treatment probes, etc.). A steering mechanism of the bronchoscope may be used to effect a deflection of a distal tip of the bronchoscope tube in one or more directions such that the distal tip of the bronchoscope may be maneuvered and approximated toward target tissue.

Typically, during a procedure, a clinician holds the bronchoscope handle with one hand and the bronchoscope tube with the other hand, and manipulates the distal tip of the bronchoscope inside the lung by rotating a deflection lever of the handle and by pushing and pulling the tube of the bronchoscope. Once the distal tip is disposed adjacent target tissue, an instrument may be inserted into the working channel of the bronchoscope to perform a diagnostic or therapeutic procedure. In some situations, an extendable working channel ("EWC") is inserted into and through the working channel of the bronchoscope. The EWC has a smaller diameter than the bronchoscope tube permitting access to more remote areas of the lung (e.g., the periphery of the lung), and defines a working channel or lumen therethrough for the passage of instruments.

The EWC is limited in the number of surgical tools it can accommodate, thus requiring the removal of one surgical tool from the EWC prior to using another surgical tool. Accordingly, there is a need for the ability to use additional surgical tools with the bronchoscope when the lumen or lumens of the bronchoscope are occupied.

### SUMMARY

Provided in accordance with the present disclosure is a surgical apparatus for use with an endoscope. The surgical apparatus includes an elastomeric sheath defining a first lumen and a second lumen. The first lumen is configured to receive an endoscope and the second lumen is configured to receive a surgical tool. The elastomeric sheath includes a navigation sensor disposed on a distal portion thereof.

In some embodiments, the navigation sensor may be an electromagnetic sensor configured to be detectable in an electromagnetic field. The surgical tool may also include a navigation sensor disposed on a distal portion thereof.

It is contemplated that the first lumen and the second lumen are separated by a wall formed within the elastomeric sheath. The inner surface of the elastomeric sheath, including the first lumen or the second lumen, may be fabricated from or coated with a lubricious material.

In is envisioned that the position of the endoscope may be fixed relative to the position of the surgical tool along a longitudinal axis defined by the elastomeric sheath.

In some embodiments, the endoscope includes at least one of a light source, a camera, or a working channel configured to receive an additional surgical tool. The surgical tool may also be a catheter configured to provide at least one of aspiration or suction.

In an additional embodiment, the surgical apparatus further includes a balloon anchor disposed on a distal portion of the elastomeric sheath. The balloon anchor may be configured to receive at least one of air or fluid to inflate the balloon anchor.

In some embodiments, the surgical apparatus further includes a collapsible lumen coupled to an outer surface of the elastomeric sheath. The collapsible lumen may be configured to receive an additional surgical tool, wherein the collapsible lumen expands when it receives the additional surgical tool. The collapsible lumen may be fabricated from an elastomeric material.

Provided in accordance with the present disclosure is a surgical apparatus for use in an endoscopy, or more particularly a bronchoscopy. The surgical apparatus includes an elastomeric sheath and a collapsible lumen coupled to an outer surface of the elastomeric sheath. The elastomeric sheath may be configured to receive an endoscope. The elastomeric sheath may further include a navigation sensor disposed on a distal portion thereof.

In some embodiments, the navigation sensor may be an electromagnetic sensor configured to be detectable in an electromagnetic field.

It is contemplated that the collapsible lumen is configured to receive a surgical tool, wherein the collapsible lumen expands when it receives a surgical tool. The surgical tool may also include a navigation sensor disposed on a distal portion thereof.

It is envisioned that the collapsible lumen may be composed on an elastomeric material.

In some embodiments, the inner surface of the elastomeric sheath is fabricated from, or coated with, a lubricious material.

It is also contemplated that the position of the endoscope is fixed along a longitudinal axis defined by the elastomeric sheath. The endoscope may also include at least one of a light source, a camera, or a working channel configured to receive an additional surgical tool.

### BRIEF DESCRIPTION OF THE DRAWINGS

Various embodiments of the present disclosure are described herein with reference to the drawings wherein:
FIG. 1 is a perspective view of a bronchoscopy system in accordance with the present disclosure;
FIG. 2A is a perspective, partial view, which depicts a distal portion of a sheath configured to receive a bronchoscope and a surgical tool in accordance with one embodiment of the present disclosure;
FIG. 2B is a partial, perspective view, which depicts a distal portion of a sheath configured to receive a bronchoscope and a surgical tool in accordance with another embodiment of the present disclosure;
FIG. 2C is a partial, perspective view, which depicts a distal portion of a sheath configured to receive a bronchoscope and a surgical tool in accordance with another embodiment of the present disclosure;
FIG. 2D is a partial, perspective view, which depicts a distal portion of a sheath configured to receive a bronchoscope and a surgical tool in accordance with yet another embodiment of the present disclosure;
FIG. 3A is a partial, perspective view, which depicts a distal portion of a sheath as shown in FIGS. 2A-2D configured with a distal balloon anchor in an unexpanded configuration;
FIG. 3B is a partial, perspective view, which illustrates an aspect of the embodiment of the sheath shown in FIG. 3A in an expanded configuration;
FIG. 4A is a partial, perspective view, which depicts a sheath as shown in FIGS. 2A-2D including an outer collapsible lumen in a collapsed configuration;
FIG. 4B is a partial, perspective view, which depicts the outer collapsible lumen of FIG. 4A in an un-collapsed configuration; and
FIG. 4C is a partial, perspective view, which depicts a distal portion of another embodiment of a sheath as shown in FIGS. 2A-2D including an outer collapsible lumen in an un-collapsed configuration.

### DETAILED DESCRIPTION

Particular embodiments of the present disclosure are described below with reference to the accompanying drawings.

The present disclosure relates to a sheath configured to receive one or more surgical tools. As will be described in further detail below, the sheath defines one or more lumens, wherein one of the lumens is configured to receive an endoscope and another of the one or more lumens is configured to receive a surgical tool. The sheath is formed from an elastomeric material and includes an electromagnetic sensor disposed at a distal portion of the sheath. The electromagnetic sensor enables the detection of the location of the electromagnetic sensor within an electromagnetic field. For illustration purposes, example embodiments depicted herein are described in the context of bronchoscopy performed by way of a bronchoscope 108. However, aspects of the present disclosure are similarly applicable to other types of endoscopy performed by way of other types of endoscopes, such as cystoscopies, nephroscopies, arthroscopies, colonoscopies, laparoscopies, and/or the like.

Embodiments of the present disclosure will now be described in detail with reference to the drawings in which like reference numerals designate identical or corresponding elements in each of the several views. As is understood in the art, the term "clinician" refers to a doctor, a physician, a nurse, a bronchoscopist, or any other care provider or support personnel. Further, as is understood in the art the term "proximal" refers to the portion of the surgical system including the coupling devices thereof, or any component thereof, that is closest to the clinician and the term "distal" refers to the portion of the surgical system including the coupling devices thereof, or any component thereof, that is furthest from the clinician.

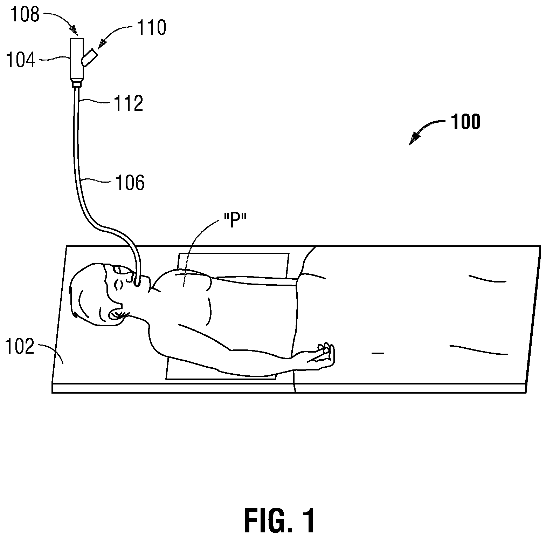

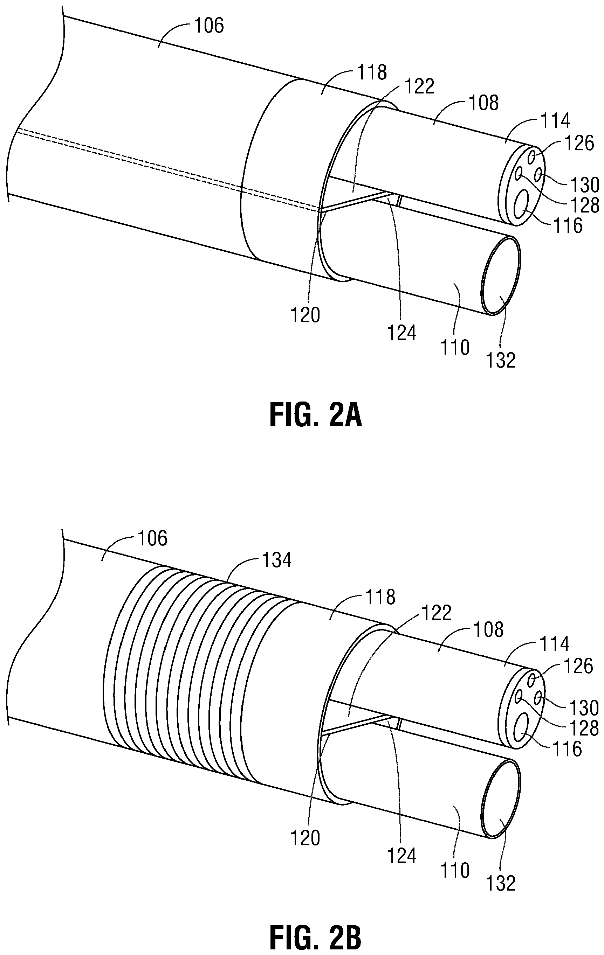

As previously stated, the embodiments herein are described in the context of bronchoscopy performed by way of a bronchoscope 108; however, it is contemplated that embodiments of the present disclosure may also be applicable to various other types of endoscopes. With reference to FIG. 1, a surgical system 100 for use in a bronchoscopy in accordance with an embodiment of the present disclosure generally includes a procedure table 102, a coupling device 104, and a sheath 106. Coupling device 104 is configured to receive a bronchoscope 108 and a surgical tool 110 (shown in greater detail in FIGS. 2A-2D). As shown in FIG. 1, sheath 106, which receives the bronchoscope 108, is coupled to a coupling device 104 at a proximal portion 112 of the sheath 106. The coupling device 104 allows for the sheath 106 to couple to various medical instruments, e.g. a bronchoscope 108 and surgical tool 110. As shown in greater detail in FIGS. 2A-2D, the sheath 106 is configured to receive a bronchoscope 108 and a surgical tool 110 (e.g., a catheter, a stapler, an ablation instrument, an ultrasonic tool, or a biopsy tool) via the coupling device 104 at a proximal portion 112 of the sheath 106. As is understood in the art, during a procedure a patient "P" is maintained on procedure table 102, permitting the clinician to insert the distal end (not explicitly shown) of the bronchoscope 108 into a natural opening or artificial incision of the patient and navigate the bronchoscope 108 to a target tissue.

In an exemplary procedure, a patient "P" is positioned on a procedure table 102 permitting a clinician to insert the distal portion 114 (shown in FIG. 2A) of the bronchoscope 108 and surgical tool 110, via the sheath 106, into a natural opening (e.g., the mouth) or artificial incision in the patient. Through articulation of the bronchoscope 108, the distal portion 114 of the bronchoscope 108 may be directed and steered within the patient "P" towards target tissue. Once the distal portion 114 of the bronchoscope 108 is brought into proximity to target tissue, or alternatively, once the distal portion 114 of the bronchoscope 108 is no longer capable of traversing through the airway of the patient "P" (e.g., due to the dimensions of the bronchoscope 108 exceeding that of the airway), an extended working channel (EWC) (not shown) may be utilized to extend the reach of the bronchoscope 108 to allow access to remote areas of the lung. In particular, the EWC is inserted through the working channel 116 (depicted in FIGS. 2A-2D) of the bronchoscope 108 and translated distally such that a distal portion of the EWC extends distally of the distal portion 114 of the bronchoscope 108. A surgical tool, for example, a microwave ablation device may be passed through the EWC and into the surgical site to treat the target tissue.

With reference to FIGS. 2A-2D, various embodiments of the sheath 106 of the surgical system 100 are described. As depicted in FIG. 2A, sheath 106 includes distal portion 118 and a wall 120 defining a first lumen 122 and a second lumen 124 within the sheath 106. The first lumen 122 is configured to receive a bronchoscope 114 and the second lumen 124 is configured to receive a surgical tool 110. Bronchoscope 114 includes a working channel 116, a camera 126, a light source 128, and a fluid channel 130. Surgical tool 110 may be a catheter and may define a third lumen 132. Surgical tool 110 may be any number of surgical instruments including a stapler, an ablation instrument, an ultrasonic tool, or a biopsy tool.

The sheath 106 may be dimensioned to be passed into various airways of the lung. In some embodiments, the sheath 106 may be dimensioned to be passed through the larger airways, such as, for example, the larynx, the trachea, and/or the bronchi. The sheath 106 may have a diameter between about 4 mm and about 6 mm such that the first lumen 122 and the second lumen 124, as defined by the wall 120 and the sheath 106, are dimensioned to receive a bronchoscope 108 or surgical tool 110 with a diameter between about 2 mm and about 3 mm (e.g. a slim bronchoscope).

The sheath 106 is composed of a flexible elastomeric material. An elastomeric material allows the sheath 106 to conform to the shape of the bronchoscope 108 and the surgical tool 110 received within the sheath 106. Wall 120 may also be composed of a flexible elastomeric material or, in another embodiment, the wall 120 may be rigid or semi-rigid to still be able to maneuver a patient's airways. In an embodiment, the bronchoscope 108 is fixed within the first lumen 122 of the sheath 106. In another embodiment, the inner surface of the first lumen 122 is composed of a material with a high coefficient of friction to inhibit movement of the bronchoscope 108. Additionally, the elastomeric sheath 106 may tightly conform to the bronchoscope 108 to limit movement of the bronchoscope 108. Conversely, in order to facilitate the passage of the surgical tool 110 through the second lumen 124, the surface of the second lumen 124 may be composed of or coated with a lubricious material (e.g. Polytetrafluoroethylene). This enables the surgical tool 110 to slide or glide within the second lumen 124 to allow for the surgical tool 110 to easily be manipulated by a clinician and extend distally past the distal portion 118 of the sheath 106 or retract back towards a proximal portion 112 of the sheath 106.

In embodiments, the distal portion 118 of the sheath 106 is rigid to provide structural support to the distal portion 118 of the sheath 106. The proximal end 112 of the sheath may couple to a coupling device 104 (depicted in FIG. 1). The coupling device 104 allows the sheath 106 to couple to a surgical device, such as a bronchoscope 108 or surgical tool 110. An example coupling device is depicted in U.S. Patent Application No. 15/606,120 (Attorney Docket No.: 1988-305), filed concurrently herewith, the entire contents of which are hereby incorporated by reference.

Referring now to FIG. 2B, in one embodiment, the sheath 106 includes a navigational sensor or electromagnetic (EM) sensor 134. In this configuration, the sheath's position within an electromagnetic field can be determined. Thus, EM sensor 134, in conjunction with an electromagnetic field generator (not shown), may be used to determine the location of the distal portion 118 of the sheath 106 within the volume of the patient's airway and chest volume based on a determined position of the EM sensor 134. A six degrees-of-freedom electromagnetic tracking system, (similar to those disclosed in U.S. Patent No. 6,188,355 and published PCT Application Nos. WO 00/10456 and WO 01/67035, the entire contents of each of which is incorporated herein by reference, or any other suitable positioning measuring system), or any other suitable positioning measuring system, may be utilized for performing navigation, although other configurations are also contemplated. One such system which utilizes these sensors is the ILOGIC® ELECTROMAGNETIC NAVIGATION BRONCHOSCOPY® (ENB™) system currently sold by Medtronic plc. The details of such a system are described in the commonly assigned U.S. Patent No. 7,233,820, filed on March 29, 2004, to Gilboa and entitled "ENDOSCOPE STRUCTURES AND TECHNIQUES FOR NAVIGATING TO A TARGET IN BRANCHED STRUCTURE," the contents of which are incorporated herein by reference. The EM sensor 134 may be in communication with a control system that provides a real-time image of the position of the EM sensor 134 of the sheath 106 within the patient's airways. Thus, by utilizing a sheath 106 equipped with an EM sensor 134, the location of the bronchoscope 108 received within the sheath 106 can be tracked. Alternative methods of tracking the location of the bronchoscope 108 typically involve utilizing a navigable catheter disposed within the working channel 116 of the bronchoscope 108. However, sheath 106 coupled with an EM sensor 134 allows for tracking of the bronchoscope 108 while leaving working channel 116 open to receive additional surgical tools. Additionally, sheath 106 with an EM sensor 134 allows for various surgical tools (i.e. bronchoscope 108 and surgical tool 110) to be adapted to include navigation capabilities.

In accordance with one aspect of the present disclosure, the EM sensor 134 may be embedded in the sheath 106 or printed directly on the sheath 106. In another embodiment, the EM sensor 134 is printed on a flexible circuit. Various known techniques may be employed to print the EM sensor 134 onto the sheath 106 including those described in commonly owned U.S. Patent Application No. 15/147,273 (Attorney Docket No. 355902.USU1 (1988-172)), filed on May 5, 2016, to Crowley et al. and U.S. Patent Application Publication No. 14/919,950 (Attorney Docket No. H-IL-00150 PRO (1988-150)), filed on December 22, 2014, to Greenburg et al., both entitled "MEDICAL INSTRUMENT WITH SENSOR FOR USE IN A SYSTEM AND METHOD FOR ELECTROMAGNETIC NAVIGATION," the entire contents of which are hereby incorporated by reference.

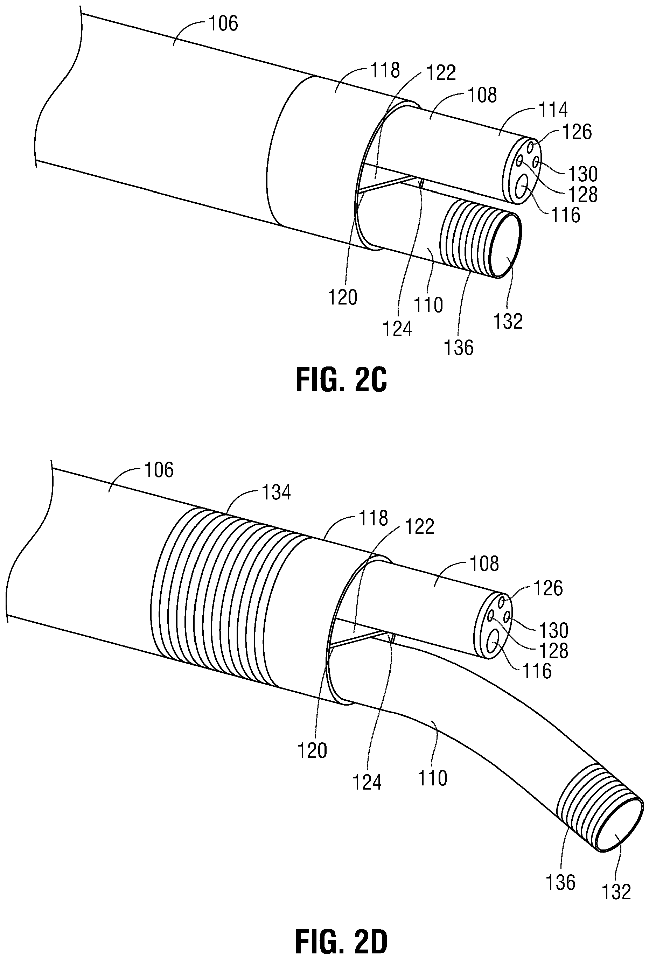

Referring now to FIGS. 2C and 2D, various other configurations of an EM sensor 134, 136 are envisioned. In particular, as shown in FIG. 2C, an EM sensor 136 is disposed on a distal portion of the surgical tool 110. Alternatively, an EM sensor 134, 136 may be incorporated on both the sheath 106 and the surgical tool 110, as shown in FIG. 2D. In this embodiment, having an EM sensor 136 on the surgical tool 110 allows for tracking of the surgical tool 110 when the surgical tool 110 is extended past the distal portion 118 of the sheath 106, as depicted in FIG. 2D. In yet another embodiment, it is envisioned that the EM sensor 136 is disposed on a distal portion 114 of the bronchoscope 108 (not shown).

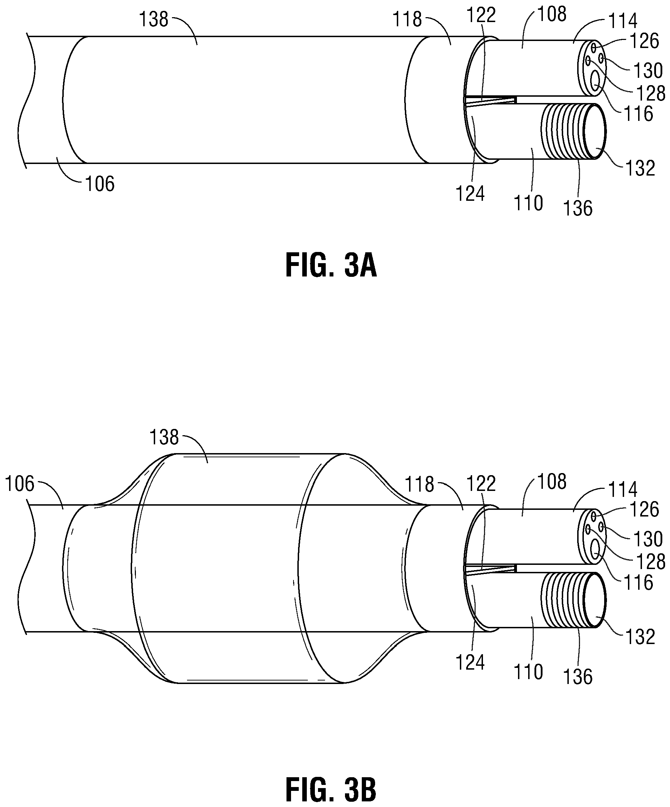

Referring now to FIGS. 3A and 3B, another envisioned embodiment of the sheath 106 includes a balloon anchor 138 disposed on the distal portion 118 of the sheath 106. The balloon anchor 138 is coupled to an external pump or syringe (not shown) and configured to be filled with either a gas (e.g. compressed air, oxygen, or nitrogen) or a fluid (e.g. water or saline). When the sheath 106 is inserted into an airway, it may be necessary to anchor the sheath 106 in a fixed location in order to allow the surgical tool 110 to be advanced further past the distal portion 118 of sheath 106. When filled, balloon 138 expands (as shown in FIG. 3B) to wedge the sheath 106 within an airway or lumen of the patient allowing for easier manipulation of the surgical tool 110 or bronchoscope 108.

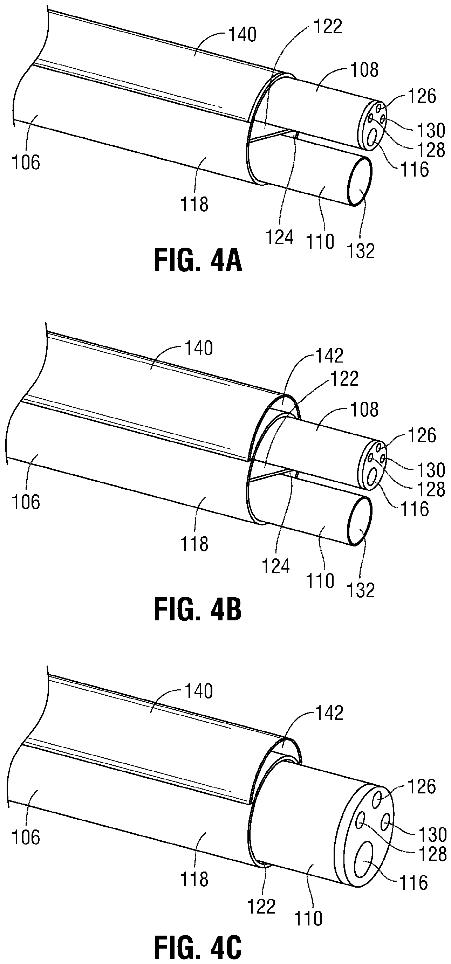

Referring now to FIGS. 4A-4C, additional embodiments of the sheath 106 include a collapsible lumen 140. Similar to the sheath 106 described above, the collapsible lumen 140 provides an additional lumen 142 to guide necessary surgical tools and devices to a target and allows for multiple tool exchanges. Collapsible lumen 140 may be placed on an exterior surface of the sheath 106 and form an additional lumen 142 separate from the sheath 106. Collapsible lumen 140 may be formed of a flexible elastomeric material or any similar biocompatible material. When in a collapsed configuration, as shown in FIG. 4A, the collapsible lumen 140 adds minimal thickness to the overall profile of the sheath 106. A surgical tool or device can be inserted into an opening at the proximal end (not shown) of the collapsible lumen 140. The force of feeding the surgical tool or device causes the collapsible lumen 140 to expand and conform to the shape of the surgical tool or device. Even in an un-collapsed configuration (shown in FIG. 4B), the collapsible lumen 140 adds minimal thickness to the overall profile of the sheath 106. FIG. 4C depicts an alternative embodiment including a sheath 106 defining the first lumen 122 configured to receive a bronchoscope 108 and a collapsible lumen 140 defining an additional lumen 142. In this embodiment, sheath 106 with collapsible lumen 140 allows a bronchoscope 108 to be modified to include an additional lumen 142 for receiving an additional surgical tool without adding significant thickness to the overall profile of the bronchoscope 108.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.
The invention may be described by reference to the following numbered paragraphs:-
1. A surgical apparatus comprising:
   an elastomeric sheath defining a first lumen configured to receive an endoscope and a second lumen configured to receive a surgical tool; and
   a navigation sensor disposed on a distal portion of the elastomeric sheath.
2. The surgical apparatus according to paragraph 1, wherein the navigation sensor is an electromagnetic sensor configured to be detectable in an electromagnetic field.
3. The surgical apparatus according to paragraph 1, wherein the surgical tool includes a navigation sensor disposed on a distal portion thereof.
4. The surgical apparatus according to paragraph 1, wherein the first lumen and the second lumen are separated by a wall formed within the elastomeric sheath.
5. The surgical apparatus according to paragraph 1, wherein the elastomeric sheath has a lubricious inner surface.
6. The surgical apparatus according to paragraph 1, wherein the position of the endoscope is fixed relative to the position of the surgical tool along a longitudinal axis defined by the elastomeric sheath.
7. The surgical apparatus according to paragraph 1, wherein the endoscope includes at least one of a light source, a camera, or a working channel configured to receive an additional surgical tool.
8. The surgical apparatus according to paragraph 1, wherein the surgical tool is a catheter configured to provide at least one of aspiration or suction.
9. The surgical apparatus according to paragraph 1, further comprising a balloon anchor disposed on a distal portion of the elastomeric sheath.
10. The surgical apparatus according to paragraph 9, wherein the balloon anchor is configured to receive at least one of air or fluid to inflate the balloon anchor.
11. The surgical apparatus according to paragraph 1, further comprising a collapsible lumen coupled to an outer surface of the elastomeric sheath.
12. The surgical apparatus according to paragraph 11, wherein the collapsible lumen is configured to receive an additional surgical tool, wherein the collapsible lumen expands when it receives the additional surgical tool.
13. The surgical apparatus according to paragraph 11, wherein the collapsible lumen is composed of an elastomeric material.

## Claims

1. A surgical apparatus comprising:
an elastomeric sheath defining a first lumen configured to receive an endoscope and a second lumen configured to receive a surgical tool; and
a navigation sensor disposed on a distal portion of the elastomeric sheath.

2. The surgical apparatus according to claim 1, wherein the navigation sensor is an electromagnetic sensor configured to be detectable in an electromagnetic field.

3. The surgical apparatus according to claim 1 or claim 2, wherein the surgical tool includes a navigation sensor disposed on a distal portion thereof.

4. The surgical apparatus according to any preceding claim, wherein the first lumen and the second lumen are separated by a wall formed within the elastomeric sheath.

5. The surgical apparatus according to any preceding claim, wherein the elastomeric sheath has a lubricious inner surface.

6. The surgical apparatus according to any preceding claim, wherein the position of the endoscope is fixed relative to the position of the surgical tool along a longitudinal axis defined by the elastomeric sheath.

7. The surgical apparatus according to any preceding claim, wherein the endoscope includes at least one of a light source, a camera, or a working channel configured to receive an additional surgical tool.

8. The surgical apparatus according to any preceding claim, wherein the surgical tool is a catheter configured to provide at least one of aspiration or suction.

9. The surgical apparatus according to any preceding claim, further comprising a balloon anchor disposed on a distal portion of the elastomeric sheath.

10. The surgical apparatus according to claim 9, wherein the balloon anchor is configured to receive at least one of air or fluid to inflate the balloon anchor.

11. The surgical apparatus according to any preceding claim, further comprising a collapsible lumen coupled to an outer surface of the elastomeric sheath.

12. The surgical apparatus according to claim 11, wherein the collapsible lumen is configured to receive an additional surgical tool, wherein the collapsible lumen expands when it receives the additional surgical tool.

13. The surgical apparatus according to claim 11 or claim 12, wherein the collapsible lumen is composed of an elastomeric material.
